# EUROPEAN PATENT APPLICATION

(11) **EP 2 371 353 A1**
(43) Date of publication of application: **05.10.2011**
(21) Application number: 11002554.1
(22) Date of filing: 28.03.2011
(51) Int. Cl.: A61K 9/00, A61K 9/70, A61K 39/36, A61K 47/26

(54) **Stabilized pharmaceutical composition, liquid preparation of stabilized pharmaceutical composition, film-form preparation, and method for producing film-form preparation**

(30) Priority: 30.03.2010 JP 2010079431
(71) Applicant: Nitto Denko Corporation, Ibaraki-shi Osaka 567-8680 (JP)
(72) Inventor: Asari, Daisuke, Ibaraki-shi Osaka 567-8680 (JP); Hori, Mitsuhiko, Ibaraki-shi Osaka 567-8680 (JP); Shishido, Takuya, Ibaraki-shi Osaka 567-8680 (JP)
(74) Representative: Müller-Boré & Partner Patentanwälte

(57) **Abstract**

The present invention provides a stabilized pharmaceutical composition that enables the production of a preparation for desensitization therapy useful as an agent for the prevention or treatment of allergic diseases such as pollen hypersensitivity, has outstanding allergen protein stability, and is useful for storage and transfer.

More specifically, the present invention provides a stabilized pharmaceutical composition containing an allergen protein and at least one type of stabilizer selected from the group consisting of sugars, sugar alcohols, and sugar fatty acids.

## Description

### TECHNICAL FIELD

The present invention relates to a stabilized pharmaceutical composition useful as an agent for the prevention or treatment of pollen hypersensitivity or the like, and applicable to desensitization therapy. More specifically, the present invention relates to a stabilized pharmaceutical composition having outstanding stability of the Cedar pollen allergen protein, and outstanding convenience in terms of storage, handling and the like.

### BACKGROUND ART

In recent years the number of patients with allergic diseases has increased as the result of changes in lifestyle and living environment, and the number of patients with allergic diseases such as pollen hypersensitivity in particular has increased.
In the case of biopharmaceuticals for the treatment of such allergic diseases, it is essential that the protein serving as the drug be stably preserved, i.e., that any loss in the biological activity thereof be held to a minimum, when it is made into a pharmaceutical product.
In drug compositions used for producing conventional biopharmaceutical products, however, stable long-term storage of the protein serving as the drug has been almost impossible.

At present almost all therapy for pollen allergies and other allergic diseases consists of symptomatic treatment with antihistamines, but recently desensitization therapy has gained attention as a mode of therapy that can be completely cured cause of an allergic disease.
Because desensitization therapy generally requires long-term administration of approximately 2 to 3 years, a dosage form that will more greatly improve the quality of life (QOL) of both caregivers and patients is needed.

At present, almost all the dosage forms used in specific desensitization therapy are injectables intended for subcutaneous injection.
However, the following problems occur with specific desensitization therapy utilizing subcutaneous injections: there is a danger of anaphylactic shock, administration must be made by a healthcare provider, the patient must make frequent visits to the healthcare provider over a long period of time, there is pain associated with the injection, and the injectable preparations must be stored under refrigeration.

In contrast, liquid and tablet preparations for sublingual administration have been marketed in Europe and the United States in recent years, and they have garnered attention because they cause few adverse reactions and they are easy to use.
There have been problems with specific desensitization therapy utilizing sublingual administration of liquid preparations, however, because the dose is imprecise, the preparations require storage under refrigeration, and the like.
In addition, there have been problems with specific desensitization therapy utilizing sublingual administration of a tablet, because the patient can swallow the tablet by mistake, it is difficult to fine-tune the dose, the portability is poor, the residue causes an unpleasant sensation in the mouth, and the like.

Moreover, in recent years film-form preparations that exhibit rapid dissolution in the mouth have been touted as a dosage form used in specific desensitization therapy.
A fast-dispersing, non-compressed solid dosage form comprising a matrix and at least one allergen has been proposed as a pharmaceutical with such a dosage form, (Patent Document 1).
However, a film-form preparation containing D-mannitol is neither disclosed nor suggested in that document, and a stabilized composition containing at least 7 wt% of water also is not disclosed therein.

In addition, film-form preparations containing an allergen and a sugar or sugar alcohol have been proposed (Patent Document 2, Patent Document 3, Patent Document 4).
However, the use of a sugar or sugar alcohol as a stabilizer is not disclosed therein.

Among the prior art film-form preparations containing such an allergen, those having the active drug dispersed or dissolved in some kind of water-soluble polymer, and those containing a non-reducing sugar and sugar alcohol have also been reported.
However, those preparations use water or a water mixture as the solvent, and the sugar or sugar alcohol is in a dissolved or recrystallized state. As a result, prior art film-form preparations containing an allergen are unsatisfactory with regard to sufficient film strength, reduction of the gummy sensation in the mouth due to the water-soluble polymer, a sticky feeling when touched by the fingers, and the like.

### PRIOR ART DOCUMENT

### PATENT DOCUMENT

Patent Document 1: JP-T 2006-513269
Patent Document 2: JP-T 2005-511522
Patent Document 3: JP-T 2007-500252
Patent Document 4: JP-T 2009-507854

### SUMMARY OF THE INVENTION

### PROBLEMS TO BE SOLVED BY THE INVENTION

In light of the above, an object of the present invention is to provide a stabilized pharmaceutical composition that enables the production of a preparation for desensitization therapy useful as an agent for the prevention or treatment of allergic diseases such as pollen hypersensitivity, has outstanding allergen protein stability, and is useful for storage and transfer; a liquid preparation of a stabilized pharmaceutical composition using the above stabilized pharmaceutical composition; a film-form preparation; and a method for producing the film-form preparation.

### MEANS FOR SOLVING THE PROBLEMS

After thorough and incisive investigation of the above problem, the inventors discovered that surprisingly an allergen protein can be stored stably by adding at least one type of stabilizer selected from the group consisting of sugars, sugar alcohols and sugar fatty acids to a drug composition containing an allergen protein such as the Cedar pollen allergen, and that this makes possible storage and transfer properties superior to prior art preparations, thus completing the present invention.
In addition, the inventors conducted diligent research concerning such a stabilized pharmaceutical composition, and as a result they discovered that when a liquid preparation and a film-form preparation were prepared using the above stabilized pharmaceutical composition, controlling the water content in the stabilized pharmaceutical composition to specified conditions, and selecting and using a specific stabilizer were effective in stabilizing the allergen protein, thus completing the present invention.
In this description the term "stabilized" in the stabilized pharmaceutical composition refers to the fact that after storage for a certain period of time after production (e.g., one to two weeks after production), or after a heat treatment when the composition is made into a film-form preparation, the activity of the allergen is greater than that in a preparation to which the stabilizer is not added (i.e., greater than 100%), and preferably at least 120% thereof.

In other words, the present invention is a stabilized pharmaceutical composition containing an allergen protein, and at least one type of stabilizer selected from the group consisting of sugars, sugar alcohols, and sugar fatty acids.
Preferably, the stabilized pharmaceutical composition of the present invention further contains water, and the stabilizer includes at least one type selected from the group consisting of D-sorbitol, isomalt, sucrose, raffinose, sorbitan fatty acid esters, and sucrose fatty acid esters.
In addition, the stabilized pharmaceutical composition of the present invention preferably contains essentially no water, and the stabilizer includes D-mannitol.
In addition, the above allergen protein preferably includes the Cedar pollen allergen protein.

The present invention is also a liquid preparation of a stabilized pharmaceutical composition obtained using a stabilized pharmaceutical composition that contains an allergen protein and at least one type of stabilizer selected from the group consisting of sugars, sugar alcohols, and sugar fatty acids; and further contains water; wherein the above stabilizer includes at least one type selected from the group consisting of D-sorbitol, isomalt, sucrose, sorbitan fatty acid esters, and sucrose fatty acid esters.
The present invention is also a film-form preparation obtained using a stabilized pharmaceutical composition that contains an allergen protein and at least one type of stabilizer selected from the group consisting of sugars, sugar alcohols, and sugar fatty acids; and contains essentially no water; wherein the above stabilizer includes D-mannitol; and the stabilized pharmaceutical composition further contains a polysaccharide.
Preferably, the above polysaccharide includes hydroxypropyl cellulose.
The present invention is also a method for producing a film-form preparation obtained by: preparing a liquid dispersion containing an allergen protein, hydroxypropyl cellulose, D-mannitol, and a polar organic solvent; forming a thin film of the liquid dispersion; and drying the thin film.
The present invention is described in detail below.

The stabilized pharmaceutical composition of the present invention contains an allergen protein and at least one type of stabilizer selected from the group consisting of sugars, sugar alcohols, and sugar fatty acids.
The above allergen protein refers to an antigen with which an antibody of a person with an allergic disease specifically reacts.
Specific examples of the above allergen protein include, for example, allergens in the pollen of trees (golden acacia, red alder, white ash, American beech, birch, box elder, mountain cedar, red cedar, common cottonwood, cypress, American elm, Chinese elm, Japanese Douglas fir, sweet gum, eucalyptus, hackberry, hickory, linden, sugar maple, mesquite, mulberry, oak, olive, pecan tree, pepper tree, pine, common privet, Russian olive, American sycamore, tree of heaven, black walnut, black willow, etc.); allergens in the pollen of grasses (cotton, Bermuda grass, Kentucky bluegrass, smooth brome, cultivated corn, meadow fescue, Johnson grass, cultivated oats, orchard grass, redtop, perennial rye grass, rice, sweet vernal grass, timothy, careless weed, pigweed, common cocklebur, sorrel dock, goldenrod, kochia, lamb's quarters, marigold, nettle, pigwood, English plantain, giant ragweed, short ragweed, western ragweed, Russian thistle, sagebrush, Scotch broom, sheep sorrel, etc.); allergens originating in insects (silkworm, mite, honeybee, wasp, ant, cockroach, etc.); allergens originating in fungi (*Alternaria tenuis, Aspergillus fumigatus*, *Botrytis cinerea, Candida albicans, Cephalosporium acremonium, Curvularia spicifera, Epicoccum nigrum, Epidermophyton floccosum, Fusarium vasinfectum, Helminthosporium interseminatum*, *Hormodendrum cladosporioides, Mucor rasemosus, Penicillium notatum*, *Phoma herbarium, Pullularia pullulans, Rhizopus nigricans*, etc.); allergens originating in the skin and hair of animals (dog, cat, bird, etc.); allergens originating in house dust; and allergens originating from foods; etc. There is no particular limitation on the allergen provided it is an antigen with which an antibody of an individual with an allergic disease specifically reacts.
To adequately provide the advantages of the present invention, a plant pollen allergen protein, e.g., Cedar or cypress allergen pollen, is preferred. Cedar group 1 and group 2 allergens, e.g., Cryj1 and Cryj2, are more preferred, and Cryj1 is even more preferred.

The content of the allergen protein will differ depending on the properties thereof, but preferably it will normally range from 1×10⁻¹⁰ to 60 wt% with regard to the total weight of the stabilized pharmaceutical composition of the present invention. When the content is less than 1×10⁻¹⁰ wt%, the preparation using the stabilized pharmaceutical composition of the present invention may not be suitable for desensitization therapy, and when the content exceeds 60 wt%, if the stabilized pharmaceutical composition of the present invention is used in a film-form preparation, for example, the film strength strength of the film may markedly decrease causing a problem with the shape retention thereof.

In the stabilized pharmaceutical composition of the present invention, the stabilizer is at least one type selected from the group consisting of sugars, sugar alcohols, and sugar fatty acids.
Examples of the above sugar or sugar alcohol include the sugar or sugar alcohol of the monosaccharides, disaccharides, and tri- to hexasaccharides.
Examples of monosaccharides include: aldotetroses such as erythrose and threose; aldopentoses such as ribose, lyxose, xylose, and arabinose; aldohexoses such as allose, talose, gulose, glucose, altrose, mannose, galactose, and idose; ketotetroses such as erythrulose; ketopentoses such as xylulose and ribulose; and ketohexoses such as psicose, fructose, sorbose, and tagatose. Examples of disaccharides include: α-diglucosides such as trehalose, kojibiose, nigerose, maltose, and isomaltose; β-diglucosides such as isotrehalose, sophorose, laminarabiose, cellobiose, and gentiobiose; α,β-diglucocides such as neotrehalose; and lactose, sucrose, isomaltulose (palatinose), and sucralose. An example of a trisaccharide is raffinose, etc. Examples of tri- to hexasaccharide oligosaccharides include cyclic oligosaccharides such as fructooligosaccharides, galactooligosaccharides, xylooligosaccharides, isomaltooligosaccharides, chitin oligosaccharides, chitosan oligosaccharides, oligoglucosamine, dextrins, and cyclodextrins, etc.

Examples of monosaccharide alcohols include: tetritols such as erythritol, D-threitol, and L-threitol; pentitols such as D-arabinitol and xylitol; hexitols such as D-iditol, galactitol (dulcitol), D-glucitol (sorbitol), and mannitol; and cyclitols such as inositol. Examples of disaccharide alcohols include maltitol, lactitol, and reduced palatinose (isomalt); and examples of oligosaccharides include pentaerythritol and reduced malt sugar starch syrup.
The above sugar or sugar alcohol in the stabilized pharmaceutical composition of the present invention can be optionally substituted, or one or two or more types thereof can be mixed together and used.

Examples of the above sugar fatty acid ester include sorbitan fatty acid ester, sucrose fatty acid ester, and the like.
Examples of the sorbitan fatty acid ester include sorbitan monooleate, sorbitan trioleate, sorbitan sesquioleate, sorbitan cocoate, polyoxyethylene sorbitan fatty acid esters, and the like.
Examples of the sucrose fatty acid ester include sucrose stearate, sucrose oleate, sucrose palmitate, sucrose myristate, sucrose behenate, sucrose erucate, sucrose mixed fatty acid esters, and the like.

When the stabilized pharmaceutical composition of the present invention also contains water, preferably the stabilizer includes at least one type selected from the group consisting of D-sorbitol, isomalt, sucrose, raffinose, sorbitan fatty acid esters, and sucrose fatty acid esters. When the stabilized pharmaceutical composition of the present invention contains water, using D-sorbitol, etc., as the stabilizer has the conspicuous effect of enabling prolongation of the storage period under refrigeration and enabling storage at room temperature. Moreover, it is possible to stabilize the allergen protein in solution during manufacturing and manufacturing can be facilitated thereby.
Here the expression "contains water" can vary depending on the ingredients of the stabilized pharmaceutical composition of the present invention, but it refers to cases wherein the water content is preferably at least 7.0 wt%, and more preferably at least 10.0 wt%, in relation to the total weight of the stabilized pharmaceutical composition of the present invention.

When the stabilized pharmaceutical composition of the present invention contains water and at least one type of stabilizer selected from the group consisting of D-sorbitol, isomalt, sucrose, sorbitan fatty acid esters, and sucrose fatty acid esters, it can suitably be adapted to a liquid preparation of a stabilized pharmaceutical composition such as an injectable or peroral preparation.
The present invention also comprises a liquid preparation of a stabilized pharmaceutical composition, i.e., a liquid preparation of a stabilized pharmaceutical composition containing a stabilized pharmaceutical composition that comprises an allergen protein, at least one type of stabilizer selected from the group consisting of sugars, sugar alcohols, and sugar fatty acids; further contains water; and the above stabilizer is at least one type selected from the group consisting of D-sorbitol, isomalt, sucrose, raffinose, sorbitan fatty acid esters, and sucrose fatty acid esters.

Preferably in the liquid preparation of a stabilized pharmaceutical composition of the present invention, the content of stabilizer, which is at least one type selected from the group consisting of D-sorbitol, isomalt, sucrose, raffinose, sorbitan fatty acid esters, and sucrose fatty acid esters, ranges from 1 to 80 wt% in relation to the total weight of the liquid preparation of a stabilized pharmaceutical composition. When the content is less than 1 wt%, the stability of the allergen protein may decrease, and when it exceeds 80 wt%, the effects on the properties of the liquid preparation of drug composition become quite large, and this may become a problem from a practical standpoint.
More preferably, the lower limit is 10 wt% and the upper limit is 60 wt%.

The liquid preparation of a stabilized pharmaceutical composition of the present invention contains water as described above, and if the water content satisfies the aforementioned conditions, the formulation can also contain an organic solvent such as a polar organic solvent, etc., as described below.

Such a liquid preparation of a stabilized pharmaceutical composition of the present invention can be produced, for example, by mixing the allergen protein and at least one type of stabilizer selected from the group consisting of D-sorbitol, isomalt, sucrose, raffinose, sorbitan fatty acid esters, and sucrose fatty acid esters wherein the particle size has been previously adjusted by pulverization, granulation, classification device, and the like, into the specified amount of water (or a mixed medium of water and an organic solvent), and stirring.

If the stabilized pharmaceutical composition of the present invention contains essentially no water, then preferably it will contain D-mannitol as the above stabilizer. When the stabilized pharmaceutical composition of the present invention contains essentially no water, if D-mannitol is used as the stabilizer, not only does this enable a prolongation of the storage period under refrigerated storage and enable storage at room temperature, but it also enables the degradation of the allergen protein to be suppressed, even at the high temperatures it may be exposed to during the manufacturing process, exhibiting an excellent stabilizing effect of the above allergen protein.
In this instance, the term "contains essentially no water" means a case wherein the stabilized pharmaceutical composition of the present invention contains no water at all, but it also allows for an amount of water originating from the humidity in the air and the manufacturing process to be contained such that the effect on the stabilization properties of the composition will be sufficiently small. Such a water content can vary depending on the ingredients of the stabilized pharmaceutical composition of the present invention, but the above term refers to a case wherein the water content is less than 7.0 wt% in relation to the total weight of the stabilized pharmaceutical composition of the present invention.

When the stabilized pharmaceutical composition of the present invention contains essentially no water and contains D-mannitol as the above stabilizer, the composition can be adapted to solid dosage forms such as a disintegrating tablet, film-form preparation, and the like.
In addition, when the stabilized pharmaceutical composition of the present invention contains essentially no water and contains D-mannitol as the above stabilizer, preferably it also contains a polysaccharide. Including a polysaccharide therein enables a stabilized film-form preparation to be suitably prepared using the stabilized pharmaceutical composition of the present invention that contains essentially no water.
The present invention also comprises a film-form preparation obtained using the stabilized pharmaceutical composition of the present invention that contains such a polysaccharide and contains essentially no water, i.e., a film-form preparation obtained using the stabilized pharmaceutical composition of the present invention that contains an allergen protein and at least one type of stabilizer selected from the group consisting of a sugar, sugar alcohol, and sugar fatty acid; that contains essentially no water; the stabilizer includes D-mannitol; and the composition further contains a polysaccharide.

The inventors conducted diligent research and as a result gained the knowledge disclosed hereinafter, thus completing the film-form preparation of the present invention.
More specifically, in preparing a film-form preparation using D-mannitol as a stabilizer, when purified water or a mixture of purified water and an organic solvent is used as the solvent rather than an organic solvent, the added particles of D-mannitol dissolve and greatly affect the properties of the film. To solve this problem, the content of D-mannitol must be reduced, but the content ratio of polysaccharide in relation to the total will increase, and as a result this can bring about a reduction in essential properties such as solubility in the mouth, feel of the film, quality of feel in the mouth, etc.
Therefore, the inventors prepared a film-form preparation wherein the D-mannitol is dispersed unchanged as fine particles by formulating the film in an organic solvent using a polysaccharide that is also soluble in the organic solvent and using D-mannitol fine particles as the stabilizer, and they discovered that the properties of the film were clearly superior to those of prior art products.

The film-form preparation, which is the preferred mode of the stabilized pharmaceutical composition of the present invention described above, provides the following advantages.
Specifically, in the film-form preparation of the present invention the D-mannitol is dispersed in a particulate state, and properties such as a controllable dissolution profile in the mouth, particularly sublingually, sufficient film strength, reduction of the gummy sensation in the mouth due to the polysaccharide, and feel when touched by the fingers, etc., were clearly better than those of prior art products.
In addition, uniformly dispersing the D-mannitol in the film in a particulate state makes possible a clear improvement only in the properties essential for taking the drug such as solubility in the mouth, feel of the film, quality of feel in the mouth, etc., without the loss of film properties such as tensile strength and stiffness that are necessary for manufacture.
In addition, using an organic solvent with a low boiling point during manufacture enables the preparation of a film-form preparation without affecting the allergen protein, which cannot withstand heat.

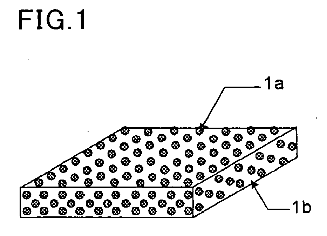

Figure 1 is a schematic drawing showing one example of an embodiment of the film-form preparation of the present invention.
As shown in Figure 1, the D-mannitol particles 1a with an average particle size of 0.1 to 100 µm are uniformly dispersed in the base material 1b that contains an edible polymer that is soluble in water and a polar organic solvent, an allergen protein, and a polysaccharide.

The thickness of the film-form preparation of the present invention is not particularly limited herein, but a thickness of 30 to 500 µm is preferred. If the thickness is less than 30 µm, the possible problems may occur in terms of film strength and handling properties of the product, and if the thickness is greater than 500 µm, the gummy sensation in the mouth originating from the edible polymer becomes stronger, and there is concern that an unpleasant sensation may occur in the mouth.
The planar shape of the film-form preparation of the present invention is not particularly limited herein, and examples include arbitrary shapes such as rectangular, square, circle, elliptical, etc.

The film-form preparation of the present invention contains the D-mannitol as disclosed above and it can also contain particles of a different sugar or sugar alcohol in a range that does not adversely affect the advantages of the present invention.
Examples of a different sugar or sugar alcohol include the monosaccharide to hexasaccharide sugars and the sugar alcohols thereof described in the above stabilized pharmaceutical composition of present invention.
Among these, a monosaccharide to trisaccharide or sugar alcohol thereof is preferred from the standpoint of the ease of dissolution in the mouth of the film-form preparation of the present invention. Furthermore, because it is thought that a sugar with reducing properties may markedly reduce antigenicity by undergoing a Maillard reaction with the allergen protein, preferably a non-reducing sugar or sugar alcohol will be used. More preferably, trehalose, xylitol, erythritol, or isomalt will be used because of their low hygroscopicity.

The average particle size of the above D-mannitol is preferably 0.1 to 100 µm in the film-form preparation of the present invention. If the average particle size exceeds 100 µm, the flexibility may not be uniform in some parts, and there will be a tendency for greater variance in the particle size in a film-form preparation of practical thickness, so the strength of the film-form preparation will tend to decrease (i.e., the film-form preparation will tend to become brittle). Conversely, if the average particle size is less than 0.1 µm, the above D-mannitol can aggregate, and similarly the flexibility of the film-form preparation may not be uniform in some places. Even more preferably, the average particle size of the above D-mannitol is 0.1 to 30 µm. By falling within this range, a liquid in which D-mannitol is uniformly dispersed can easily be prepared in a practical manufacturing process.
In addition, in this description the above average particle size refers to a Mass-median-diameter (D50) determined by a laser-scattering particle size distribution analyzer.

Further the above D-mannitol can be of any shape provided they are solids, or in a contextually suitable case, aggregates thereof within the above average particle size range. A commercially available product prepared so that the average particle size of the D-mannitol lies within the above range can be used, or a commercially available product can be used after sizing so that the average particle size lies within the above range. Adjustment of the above average particle size can be carried out by pulverization or granulation using dry granulation, wet granulation, etc., classification using a sieve, mechanical classifier, etc. To easily obtain solids lying within the range for the above average particle size, the above D-mannitol is preferably not obtained by a dissolution and recrystallization procedure.

The above D-mannitol preferably constitutes 1 to 80 wt% of the total weight of the film-form preparation of the present invention. In a film-form preparation of practical thickness, if the content is less than 1 wt%, no clear improvement is seen in the properties of rapid dissolution profile in the mouth, sufficient film strength, decrease of gummy sensation in the mouth originating in the edible polymer, and the sticky sensation when touched by the fingers. If the content exceeds 80 wt%, it is possible that problems with shape retention, etc., of the product will occur unless the size of the D-mannitol is made very small. More preferably, the lower limit for the content of the D-mannitol is 10 wt%, and more preferably the upper limit is 60 wt%. When the content lies within this range, improvement in the rapid dissolution profile in the mouth, sufficient film strength, gummy sensation in the mouth originating in the edible polymer described below, and sticky sensation when touched by the fingers becomes possible with a particle size that is practical from a manufacturing standpoint.
Many saccharides are sweet, and this is actually a favorable property for a film-form preparation that readily dissolves in the mouth. Of course, plasticizer can also be added to the film-form preparation as desired.
In addition, a stabilizer other than the above D-mannitol can be added in a range that does not inhibit the advantages of the present invention.

An edible polymer that is soluble both in water and a polar organic solvent is preferred as the polysaccharide contained in the film-form preparation of the present invention.
The edible polymer is not particularly limited herein provided it can be formed into a film, is edible, and dissolves in a polar organic solvent wherein the above D-mannitol does not dissolve.
In this description the term "edible" refers to a pharmaceutically acceptable polymer that can be administered orally.

To be more specific, polyvinyl pyrrolidone (hereinafter, "PVP") and hydroxypropylcellulose (hereinafter, "HPC") can be suitably used for the above edible polymer.
These edible polymers are sufficiently soluble in both water and a polar organic solvent, so they satisfy both conditions of rapidly dissolving in the mouth and enabling the use of a polar organic solvent during the manufacturing process of the film-form preparation of the present invention. This enables the above D-mannitol that is insoluble in a polar organic solvent to be uniformly dispersed and carried as particles in the film-form preparation.
The use of HPC as the above edible polymer is even more preferred. The hygroscopicity of HPC in relation to relative humidity is lower than that of PVP, and this is considered preferable from a practical standpoint. These materials can be used alone, or as a combination of two or more types thereof.
The time of dissolution in the mouth can be controlled by controlling the thickness of the film-form preparation of the present invention, and the time of dissolution in the mouth can be easily and intentionally controlled by suitably adjusting the molecular weight of the PVP, HPC, or other edible polymer.

The molecular weight of the PVP is preferably 2,500 to 3,000,000, and more preferably 2,500 to 1,200,000. If the molecular weight is less than 2,500, the polymer may have poor stability and hygroscopicity, and conversely, if the molecular weight exceeds 3,000,000, it may have poor solubility.

The molecular weight of the above HPC is preferably 10,000 to 1,150,000, and more preferably 10,000 to 370,000. If the molecular weight is less than 10,000, the polymer may have poor hygroscopicity and stability, and if the molecular weight exceeds 1,150,000, it may have poor solubility.
In the present description the term molecular weight refers to the weight-average molecular weight, which can be determined by gel permeation chromatography.

The amount of hydroxypropoxy group-substitution degree in the above HPC is preferably 50.0% or higher. When it is less than 50%, there is concern that the solubility thereof in water and polar organic solvents may become poor. In this case, the method of measuring the amount of hydroxypropoxy group-substitution gegree follows the quantitative method described in the section entitled "Hydroxypropyl cellulose" in the Official Monographs of the Fifteenth Edition of the Japanese Pharmacopoeia. More Preferably, the hydroxypropoxy group-substitution degree in the above HPC is at least 53.4%.

The content of the above edible polymer in the film-form preparation of the present invention is preferably 1 to 80 wt% in relation to the total molecular weight of the film-form preparation. If the polymer content is less than 1 wt%, the film-form preparation of the present invention will become brittle and not exhibit sufficient strength, and if the polymer content exceeds 80 wt%, a gummy sensation originating in the edible polymer tends to occur in the mouth. A more preferred lower limit of the content of the above edible polymer is 10 wt%, and a more preferred upper limit is 70 wt%.

In addition to the edible polymer that is soluble both in water and a polar organic solvent, a suitable amount of an edible polymer that is soluble only in water, or an edible polymer that is insoluble in both water and organic polymer (hereinafter, both such edible polymers are covered by the blanket expression "other edible polymer") can be used in combination therewith provided the content is within a range that does not hinder the effect of the present invention.
Examples of the other edible polymer include synthetic polymers such as polyvinyl alcohol, carboxyvinyl polymer, hydroxypropyl methylcellulose, hydroxyethyl cellulose, methylcellulose, ethylcellulose, low-substituted hydroxypropyl cellulose, crystalline cellulose, carboxymethylcellulose sodium, carboxymethylcellulose calcium, carboxymethylcellulose, and carboxymethyl starch sodium; and polymers obtained from natural substances such as sodium alginate, dextran, casein, pullulan, pectin, guar gum, xanthan gum, tragacanth gum, acacia gum, gum arabic, gellan gum, and starch.

To define solubility in water or the polar organic solvent in the present description, if the volume of water or polar organic solvent necessary to dissolve 1 g of solute at 20°C is 100 mL or more, the expression "insoluble" is used with respect to that solvent, and if the volume of water or polar organic solvent necessary to dissolve 1 g of solute is less than 5 mL, the expression "soluble" is used. In addition, the expression "very soluble" is used if the volume of water or polar organic solvent necessary to dissolve 1 g of solute is less than 3 mL. The solubility of the above D-mannitol particles used in the present invention is known to decrease as the temperature of the polar organic solvent increases, and by utilizing this knowledge it is possible to lower the solubility of the above D-mannitol particles even further to stabilize the same in the state of fine particles.

In addition to the above materials, the film-form preparation of the present invention can also contain a suitable amount of fragrance, flavoring, sweetener, coloring, preservative, antioxidant, stabilizer other than the above D-mannitol, surfactant, plasticizer (polyethylene glycol (PEG), etc.) as ingredients constituting the base of the film-form preparation.

The film-form preparation of the present invention having the above constitution enables self-administration by the patient without the pain accompanying an injection, and the dose can be adjusted by dividing up the film-form preparation. Moreover, the film-form preparation has excellent portability, gives no sensation of a residue, and provides excellent protection against accidental swallowing because the dosage form can easily be distinguished from a tablet. The film-form preparation is also easy for a caregiver to administer, etc., and it can greatly improve the QOL of both.patients and caregivers.
Furthermore, using a polar organic solvent as the solvent enables the film-form preparation to be dried at low temperatures, which is favorable for a preparation containing an allergen that cannot withstand high temperatures.

The film-form preparation of the present invention can be produced by the following process, for example.
Specifically, first a particle dispersion containing a specified quantity of polar organic solvent (e.g., ethanol, isopropanol, acetone, etc.), D-mannitol particles wherein the particle size has been adjusted beforehand by pulverization, granulation, a classification device, etc., and the allergen protein is prepared. Next, a suitable amount of the resulting particle dispersion is spread onto a publicly known release film to form a thin film, and the thin film is dried. Additionally, the resulting thin film is preferably cut to the desired size, sealed and packaged as needed, and made into a product.
The present invention also comprises the method for producing such a film-form preparation.

When preparing the above particle dispersion, if bubbles should form therein, the liquid can be let stand overnight or degassed under reduced pressure.
A polar organic solvent wherein the edible polymer dissolves, but the above D-mannitol does not can be used in the preparation of the above particle dispersion. A single solvent or a mixture of solvents can be used. Specifically, ethanol, propanol, and acetone are the primary solvents, and ethanol is more preferred, and purified water can be added thereto in an amount such that the D-mannitol particles do not dissolve.

### EFFECTS OF THE INVENTION

The present invention provides a stabilized pharmaceutical composition that enables the production of a preparation for desensitization therapy (a liquid preparation of a stabilized pharmaceutical composition, or a film-form preparation) that is useful for the prevention or treatment of an allergic disease such as pollen hypersensitivity, has excellent stability of the allergen protein, and is useful for storage and transfer.
The liquid preparation of a stabilized pharmaceutical composition obtained using the stabilized pharmaceutical composition of the present invention enables stability of the allergen protein to be increased over widely known liquid formulation pharmaceutical compositions. This suppresses allergen protein degradation at the high temperatures to which it may be exposed during the manufacturing process, prolongs the storage period under refrigeration, and enables storage at room temperature.
In particular, one embodiment of the present invention relating to the stabilized pharmaceutical composition that contains water provides an outstanding advantage of prolonging the storage period under refrigeration and enabling storage at room temperature.
In addition, one embodiment of the present invention relating to the stabilized pharmaceutical composition that contains essentially no water provides the outstanding advantages of not only prolonging the storage period under refrigeration and enabling storage at room temperature, but also enabling the suppression of allergen protein degradation even at the high temperatures to which it may be exposed during the manufacturing process.
The film-form preparation of the present invention that is obtained using the stabilized pharmaceutical composition of the present invention enables self-administration of an allergen by the patient without the pain accompanying an injection, and the dose can be adjusted by dividing up the film-form preparation. Moreover, the film-form preparation has excellent portability, gives no sensation of a residue, and provides excellent protection against accidental swallowing because the dosage form can easily be distinguished from a tablet. The film-form preparation is also easy for a caregiver to administer, etc., and it can greatly improve the QOL of both patients and caregivers.
Additionally the film-form preparation of the present invention contains polysaccharide and D-mannitol, and enables arbitrary control of the dissolution time in the mouth, especially sublingually by using, as the polysaccharide, an edible polymer that is soluble in both water and a polar organic solvent. In a preferred embodiment, the dissolution time can be adjusted between 2 to 300 sec, so the film-form preparation of the present invention is particularly suitable for desensitization therapy.
Moreover, the D-mannitol particles do not dissolve in the polar organic solvent. Because a polar organic solvent is used to manufacture the film-form preparation of the present invention, the D-mannitol particles can be uniformly dispersed and carried in the base of the film-form preparation in a particulate state without dissolving during the manufacturing process.
The film-form preparation of the present invention contains the above D-mannitol particles and hydroxypropyl cellulose. This not only enables the film-form preparation to have sufficient film strength, but also makes it possible to reduce the gummy sensation in the mouth originating in the edible polymer and improve the feel when touched by the fingers, and obtain a high film strength.
In addition, in the method for producing the film-form preparation of the present invention, it is possible to dry the same at a lower temperature by using a polar organic solvent as the solvent, and even when using an allergen protein that cannot withstand high temperatures, the film-form preparation can be manufactured while reducing the deleterious effects thereon.

### BRIEF DESCRIPTION OF THE DRAWINGS

Figure 1 is a schematic drawing showing one example of an embodiment of the film-form preparation of the present invention.

### MODE FOR CARRYING OUT THE INVENTION

The present invention is described in detail below through examples, but is by no means limited thereto.

### (Example 1)

First a stabilized pharmaceutical composition with a water content of 17.7 wt% was prepared by adding 55.0 parts by weight of standardized allergen therapy extract "Torii" cedar pollen 2000 JAU/mL (produced by Torii Pharmaceutical Co., Ltd.) to 100.0 parts by weight of D-sorbitol, and stirring to dissolve. The standard allergen therapy extract "Torii" cedar pollen 2000 JAU/mL contains 50 wt% of water.
This stabilized pharmaceutical composition was stored for two weeks at 30 ± 2°C, and the allergen activity was measured after one and two weeks. Table 10 shows the results.

### (Examples 2 to 4)

Stabilized pharmaceutical compositions were prepared using the same procedure as in Example 1 except the ingredients shown in Table 1 were used.
The resulting stabilized pharmaceutical compositions were stored for two weeks at 30 ± 2°C, and the allergen activity was measured after one and two weeks. Table 10 shows the results.

### (Test Example 1)

A drug composition with a water content of 17.7 wt% was prepared by adding 55.0 parts by weight of standard allergen therapy extract "Torii" cedar pollen 2000 JAU/mL (produced by Torii Pharmaceutical Co., Ltd.) to 100.0 parts by weight of erythritol, and stirring to dissolve.
This drug composition was stored for two weeks at 30 ± 2°C, and the allergen activity was measured after one and two weeks. Table 10 shows the results.

### (Test Examples 2 to 5)

Drug compositions were prepared using the same procedure as in Test Example 1 except the ingredients shown in Table 1 were used.
The resulting drug compositions were stored for two weeks at 30 ± 2°C, and the allergen activity was measured after one and two weeks. Table 10 shows the results.

**Table 1**

| Component | Formulation [part by weight] | | | | | | | | |
|---|---|---|---|---|---|---|---|---|---|
| | Example | | | | Test Example | | | | |
| | 1 | 2 | 3 | 4 | 1 | 2 | 3 | 4 | 5 |
| Standard therapeutic allergen extract Cedar pollen 2,000JAU/mL | 55 | 55 | 55 | 55 | 55 | 55 | 55 | 55 | 55 |
| D-sorbitol | 100 | - | - | - | - | - | - | - | - |
| Isomalt | - | 100 | - | - | - | - | - | - | - |
| Erythritol | - | - | - | - | 100 | - | - | - | - |
| Xylitol | - | - | - | - | - | 100 | - | - | - |
| Maltitol | - | - | - | - | - | - | 100 | - | - |
| D-mannitol | - | - | - | - | - | - | - | 100 | - |
| Sucrose | - | - | 100 | - | - | - | - | - | - |
| Raffinose | - | - | - | 100 | - | - | - | - | - |
| D-glucose | - | - | - | - | - | - | - | - | 100 |
| Water content (wt %) | 17.7 | 17.7 | 17.7 | 17.7 | 17.7 | 17.7 | 17.7 | 17.7 | 17.7 |

### (Example 5)

A stabilized pharmaceutical composition with a water content of 17.7 wt% was prepared by adding 55.0 parts by weight of standard allergen therapy extract "Torii" cedar pollen 2000 JAU/mL (produced by Torii Pharmaceutical Co., Ltd.) to 100.0 parts by weight of sucrose stearate, and stirring to dissolve.
This solution was stored for two weeks at 30 ± 2°C, and the allergen activity was measured after one and two weeks. Table 11 shows the results.

### (Examples 6 to 9)

Stabilized pharmaceutical compositions were prepared using the same procedure as in Example 5 except the ingredients shown in Table 2 were used.
The resulting stabilized pharmaceutical compositions were stored for two weeks at 30 ± 2°C, and the allergen activity was measured after one and two weeks. Table 11 shows the results.

### (Test Example 6)

A drug composition with a water content of 17.7 wt% was prepared by adding 55.0 parts by weight of standard allergen therapy extract "Torii" cedar pollen 2000 JAU/mL (produced by Torii Pharmaceutical Co., Ltd.) to 100.0 parts by weight of trehalose.
This drug composition was stored for two weeks at 30 ± 2°C, and the allergen activity was measured after one and two weeks. Table 11 shows the results.

### (Test Example 7)

A drug composition was prepared using the same procedure as in Test Example 6 except the ingredients shown in Table 2 were used.
The drug composition was stored for two weeks at 30 + 2°C, and the allergen activity was measured after one and two weeks. Table 11 shows the results.

### (Comparative Example 1)

As a comparison, 55.0 parts by weight of standard allergen therapy extract "Torii" cedar pollen 2000 JAU/mL (produced by Torii Pharmaceutical Co., Ltd.) was taken, stored for two weeks at 30 ± 2°C, and the allergen activity was measured after one and two weeks. Table 11 shows the results.

**Table 2**

| Component | Formulation [parts by weight] | | | | | | | |
|---|---|---|---|---|---|---|---|---|
| | Example | | | | | Test Example | | Comparative Example |
| | 5 | 6 | 7 | 8 | 9 | 6 | 7 | 1 |
| Standard therapeutic allergen extract Cedar pollen 2,000 JAU/mL | 55 | 55 | 55 | 55 | 55 | 55 | 55 | 55 |
| Trehalose | - | - | - | - | - | 100 | - | - |
| Sucralose | - | - | - | - | - | - | 100 | - |
| Sucrose stearic acid ester | 100 | - | - | - | - | - | - | - |
| Sorbitan trioleate | - | 100 | - | - | - | - | - | - |
| Sorbitan monooleate | - | - | 100 | - | - | - | - | - |
| Sorbitan cocoate | - | - | - | 100 | - | - | - | - |
| Sorbitan sesquioleate | - | - | - | - | 100 | - | - | - |
| Water content (wt %) | 17.7 | 17.7 | 17.7 | 17.7 | 17.7 | 17.7 | 17.7 | 17.7 |

For the sugar and sugar alcohol particles used in the following examples described as fine particles, the particle size was adjusted using a jet mill. The Mass-median-diameter (D50) was measured using a laser-scattering particle size distribution analyzer, and that value was used as the particle size index of the fine particles. Table 3 shows the Mass-median-diameter (D50) of the sugars and sugar alcohols used.
For particles not described as fine particles, a pharmaceutical additive was used without additional treatment. Isomalt A contained a 1:1 ratio (by mass) of 6-0-α-D-Glucopyranosyl-D-sorbitol (1,6-GPS) and 1-0-α-D-Glucopyranosyl-D-mannitol dehydrate (1,1-GMP) (produced by BENEO-Palatint GmbH, brand: galenIQ 800), and Isomalt B contained a 3:1 ratio (by mass) of 1,6-GPS and 1,1-GPM (BENEO-Palatint GmbH, brand: galenIQ 801).

**Table 3**

| Sugars and sugar alcohols | 50% average particle size [um] |
|---|---|
| D-mannitol particles | 2 |
| Isomalt A fine particles | 3 |
| Isomalt B fine particles | 3 |
| D-sorbitol particles | 2 |

### (Example 10)

First 1.0 part by weight of polyethylene glycol and 43.0 parts by weight of D-mannitol fine particles were added to 105.5 parts by weight of ethanol and dispersed by sonication. Next 50.0 parts by weight of HPC (produced by Nippon Soda Co., Ltd., brand name: Nisso HPC-SSL) with a molecular weight of approximately 30,000 and a hydroxypropoxy group-substitution degree of 53.4 to 77.5% was added thereto, stirred, and dissolved. Then 11.0 parts by weight of standard allergen therapy extract "Torii" cedar pollen 2000 JAU/mL (produced by Torii Pharmaceutical Co., Ltd.) was added thereto, stirred and mixed using a rolling mixer to prepare a stabilized pharmaceutical composition with a water content of 2.6 wt%.
After the stabilized pharmaceutical composition was adequately degassed, it was spread onto a polyester release film and dried for 5 min at normal temperatures, then dried by heating at 60°C for 6 min to prepare a film with a thickness of approximately 100 µm. The resulting film was cut into 5 cm² rectangles to obtain a film-form preparation.

### (Test Example 8)

First 1.0 part by weight of polyethylene glycol and 43.0 parts by weight of isomalt A fine particles were added to 105.5 parts by weight of ethanol and dispersed by sonication. Next 50.0 parts by weight of HPC (produced by Nippon Soda Co., Ltd., brand name: Nisso HPC-SSL) with a molecular weight of approximately 30,000 and a hydroxypropoxy group-substitution degree of 53.4 to 77.5% was added thereto, stirred, and dissolved. Then 11.0 parts by weight of standard allergen therapy extract "Torii" cedar pollen 2000 JAU/mL (produced by Torii Pharmaceutical Co., Ltd.) was added thereto, stirred and mixed using a rolling mixer to prepare a drug composition with a water content of 2.6 wt%.
After the drug composition was adequately degassed, it was spread onto a polyester release film and dried for 5 min at normal temperatures, then dried by heating at 60°C for 6 min to prepare a film with a thickness of approximately 100 µm. The resulting film was cut into 5 cm² rectangles to obtain a film-form preparation.

### (Test Examples 9 and 10)

film-form preparations were obtained by the same procedure as in Test Example 8 except the ingredients shown in Table 4 were used.

### (Comparative Example 2)

First 1.0 part by weight of polyethylene glycol and 93.0 parts by weight of HPC (produced by Nippon Soda Co., Ltd., brand name: Nisso HPC-SSL) with a molecular weight of approximately 30,000 and a hydroxypropoxy group-substitution degree of 53.4 to 77.5% were added to 105.5 parts by weight of ethanol, stirred, and dissolved.. Next 11.0 parts by weight of standard allergen therapy extract "Torii" cedar pollen 2000 JAU/mL (produced by Torii Pharmaceutical Co., Ltd.) was added thereto, stirred and mixed using a rolling mixer to prepare a drug composition with a water content of 2.6 wt%. After the drug composition was adequately degassed, it was spread onto a polyester release film and dried for 5 min at normal temperatures, then dried by heating at 60°C for 6 min to prepare a film with a thickness of approximately 100 µm. The resulting film was cut into 5 cm² rectangles to obtain a film-form preparation.

**Table 4**

| Component | Formulation [parts by weight] | | | | |
|---|---|---|---|---|---|
| | Example | Test Example | | | Comparative Example |
| | 10 | 8 | 9 | 10 | 2 |
| Standard therapeutic allergen extract Cedar pollen 2,000JAU/mL | 11.0 | 11.0 | 11.0 | 11.0 | 11.0 |
| HPC | 50.0 | 50.0 | 50.0 | 50.0 | 93.0 |
| Polyethylene glycol 400 | 1.0 | 1.0 | 1.0 | 1.0 | 1.0 |
| D-mannitol fine particles | 43.0 | - | - | - | - |
| Isomalt A fine particles | - | 43.0 | - | - | - |
| Isomalt B fine particles | - | - | 43.0 | - | - |
| D-sorbitol fine particles | - | - | - | 43.0 | - |
| Ethanol | 105.5 | 105.5 | 105.5 | 105.5 | 105.5 |
| Water content (wt %) | 2.6 | 2.6 | 2.6 | 2.6 | 2.6 |
| Drying time at normal temperature [min] | 5 | 5 | 5 | 5 | 5 |
| Heat drying temperature [°C] | 60 | 60 | 60 | 60 | 60 |
| Heat drying time [min] | 6 | 6 | 6 | 6 | 6 |
| Reduced-pressure drying time [min] | - | - | - | - | - |

### (Example 11)

A Film-form preparation was obtained by the same procedure as in Example 10 except the ingredients and drying conditions shown in Table 5 were used.

### (Test Examples 11 to 13)

Film-form preparations were obtained by the same procedure as in Test Example 8 except the ingredients and drying conditions shown in Table 5 were used.

### (Comparative Example 3)

A film-form preparation was obtained by the same procedure as in Comparative Example 2 except the ingredients and drying conditions shown in Table 5 were used.

**Table 5**

| Component | Formulation [parts by weight] | | | | |
|---|---|---|---|---|---|
| | Example | Test Example | | | Comparative Example |
| | 11 | 11 | 12 | 13 | 3 |
| Standard therapeutic allergen extract Cedar pollen 2,000 JAU/mL | 11.0 | 11.0 | 11.0 | 11.0 | 11.0 |
| HPC | 50.0 | 50.0 | 50.0 | 50.0 | 93.0 |
| Polyethylene glycol 400 | 1.0 | 1.0 | 1.0 | 1.0 | 1.0 |
| D-mannitol fine particles | 43.0 | - | - | - | - |
| Isomalt A fine particles | - | 43.0 | - | - | - |
| Isomalt B fine particles | - | - | 43.0 | - | - |
| D-sorbitol fine particles | - | - | - | 43.0 | - |
| Ethanol | 105.5 | 105.5 | 105.5 | 105.5 | 105.5 |
| Water content (wt %) | 2.6 | 2.6 | 2.6 | 2.6 | 2.6 |
| Drying time at room temperature [min] | 60 | 60 | 60 | 60 | 60 |
| Heat drying temperature [°C] | 60 | 60 | 60 | 60 | 60 |
| Heat drying time [min] | 6 | 6 | 6 | 6 | 6 |
| Reduced-pressure drying time [min] | - | - | - | - | - |

### (Example 12)

A film-form preparation was obtained by the same procedure as in Example 10 except the ingredients and drying conditions shown in Table 6 were used.

### (Test Examples 14 to 16)

Film-form preparations were obtained by the same procedure as in Test Example 8 except the ingredients and drying conditions shown in Table 6 were used.

### (Comparative Example 4)

A film-form preparation was obtained by the same procedure as in Comparative Example 2 except the ingredients and drying conditions shown in Table 6 were used.

**Table 6**

| Component | Formulation [parts by weight] | | | | |
|---|---|---|---|---|---|
| | Example | Test Example | | | Comparative Example |
| | 12 | 14 | 15 | 16 | 4 |
| Standard therapeutic allergen extract Cedar pollen 2,000JAU/mL | 11.0 | 11.0 | 11.0 | 11.0 | 11.0 |
| HPC | 50.0 | 50.0 | 50.0 | 50.0 | 93.0 |
| Polyethylene glycol 400 | 1.0 | 1.0 | 1.0 | 1.0 | 1.0 |
| D-mannitol fine particles | 43.0 | - | - | - | - |
| Isomalt A fine particles | - | 43.0 | - | - | - |
| Isomalt B fine particles | - | - | 43.0 | - | - |
| D-sorbitol fine particles | - | - | - | 43.0 | - |
| Ethanol | 105.5 | 105.5 | 105.5 | 105.5 | 105.5 |
| Water content (wt %) | 2.6 | 2.6 | 2.6 | 2.6 | 2.6 |
| Drying time at normal temperature [min] | - | - | - | - | - |
| Heat drying temperature [°C] | - | - | - | - | - |
| Heat drying time [min] | - | - | - | - | - |
| Reduced-pressure drying time [min] | 60 | 60 | 60 | 60 | 60 |

### (Test Examples 17 to 26)

Film-form preparations were obtained by the same procedure as in Test Example 8 except the ingredients and drying conditions shown in Table 7 were used.

**Table 7**

| Component | Formulation [parts by weight] | | | | | | | | | |
|---|---|---|---|---|---|---|---|---|---|---|
| | Example | | | | | | | | | |
| | 17 | 18 | 19 | 20 | 21 | 22 | 23 | 24 | 25 | 26 |
| Standard therapeutic allergen extract Cedar pollen 2,000 JAU/mL | 11.0 | 11.0 | 11.0 | 11.0 | 11.0 | 11.0 | 11.0 | 11.0 | 11.0 | 11.0 |
| HPC | 50.0 | 50.0 | 50.0 | 50.0 | 50.0 | 50.0 | 50.0 | 50.0 | 50.0 | 50.0 |
| Polyethylene glycol 400 | 1.0 | 1.0 | 1.0 | 1.0 | 1.0 | 1.0 | 1.0 | 1.0 | 1.0 | 1.0 |
| D-sorbitol fine particles | 43.0 | 43.0 | 43.0 | 43.0 | 43.0 | 43.0 | 43.0 | 43.0 | 43.0 | 43.0 |
| Ethanol | 105.5 | 105.5 | 105.5 | 105.5 | 105.5 | 105.5 | 105.5 | 105.5 | 105.5 | 105.5 |
| Water content (wt %) | 2.6 | 2.6 | 2.6 | 2.6 | 2.6 | 2.6 | 2.6 | 2.6 | 2.6 | 2.6 |
| Drying time at normal temperature [min] | 5 | 5 | 5 | 5 | 5 | 5 | 5 | 5 | 5 | - |
| Heat drying temperature [°C] | 40 | 40 | 40 | 60 | 60 | 60 | 80 | 80 | 80 | - |
| Heat drying time [min] | 9 | 12 | 15 | 3 | 6 | 9 | 3 | 6 | 9 | - |
| Reduced-pressure drying time [min] | - | - | - | - | - | - | - | - | - | 60 |

### (Examples 13 to 22)

film-form preparations were obtained by the same procedure as in Example 10 except the ingredients and drying conditions shown in Table 8 were used.

**Table 8**

| Component | Formulation [parts by weight] | | | | | | | | | |
|---|---|---|---|---|---|---|---|---|---|---|
| | Example | | | | | | | | | |
| | 13 | 14 | 15 | 16 | 17 | 18 | 19 | 20 | 21 | 22 |
| Standard therapeutic allergen extract Cedar pollen 2,000 JAU/mL | 11.0 | 11.0 | 11.0 | 11.0 | 11.0 | 11.0 | 11.0 | 11.0 | 11.0 | 11.0 |
| HPC | 50.0 | 50.0 | 50.0 | 50.0 | 50.0 | 50.0 | 50.0 | 50.0 | 50.0 | 50.0 |
| Polyethylene glycol 400 | 1.0 | 1.0 | 1.0 | 1.0 | 1.0 | 1.0 | 1.0 | 1.0 | 1.0 | 1.0 |
| D-mannitol fine particles | 43.0 | 43.0 | 43.0 | 43.0 | 43.0 | 43.0 | 43.0 | 43.0 | 43.0 | 43.0 |
| Ethanol | 105.5 | 105.5 | 105.5 | 105.5 | 105.5 | 105.5 | 105.5 | 105.5 | 105.5 | 105.5 |
| Water content (wt %) | 2.6 | 2.6 | 2.6 | 2.6 | 2.6 | 2.6 | 2.6 | 2.6 | 2.6 | 2.6 |
| Drying time at normal temperature [min] | 10 | 10 | 10 | 10 | 10 | 10 | 10 | 10 | 10 | - |
| Heat drying temperature [°C] | 40 | 40 | 40 | 50 | 50 | 50 | 60 | 60 | 60 | - |
| Heat drying time [min] | 30 | 60 | 120 | 5 | 10 | 15 | 5 | 10 | 15 | - |
| Reduced-pressure drying time [min] | - | - | - | - | - | - | - | - | - | 60 |

### (Examples 23 to 33)

film-form preparations were obtained by the same procedure as in Example 10 except the ingredients and drying conditions shown in Table 9 were used.

**Table 9**

| Component | Formulation [parts by weight] | | | | | | | | | | |
|---|---|---|---|---|---|---|---|---|---|---|---|
| | Example | | | | | | | | | | |
| | 23 | 24 | 25 | 26 | 27 | 28 | 29 | 30 | 31 | 32 | 33 |
| Standard therapeutic allergen extract Cedar pollen 2.000JAU/mL | 11.0 | 11.0 | 11.0 | 11 0 | 11.0 | 11.0 | 11.0 | 11.0 | 11.0 | 11.0 | 11.0 |
| HPC | 50.0 | 50.0 | 50.0 | 50 | 500 | 50.0. | 50.0 | 50.0 | 50.0 | 50.0 | 50.0 |
| Polyethylene glycol 400 | 1.0 | 1.0 | 1.0 | 1.0 | 1.0 | 1.0 | 1.0 | 1.0 | 1.0 | 1.0 | 1.0 |
| D-mannitol fine particles | 43.0 | 43.0 | 43.0 | 43.0 | 43.0 | 43.0 | 43.0 | 43.0 | 43.0 | 43.0 | 43.0 |
| Acetone | 105.5 | 105.5 | 105.5 | 105.5 | 105.5 | 105.5 | 105.5 | 105.5 | 105.5 | 105.5 | 105.5 |
| Water content (wt %) | 2.6 | 2.6 | 2.6 | 2.6 | 2.6 | 2.6 | 2.6 | 2.6 | 2.6 | 2.6 | 2.6 |
| Drying time at normal temperature [min] | 10 | 10 | 10 | 10 | 10 | 10 | 10 | 10 | 10 | - | 1440 |
| Heat drying temperature [°C] | 40 | 40 | 40 | 50 | 50 | 50 | 60 | 60 | 60 | - | - |
| Heat drying time [min] | 10 | 15 | 20 | 5 | 10 | 15 | 5 | 10 | 15 | - | - |
| Reduced-pressure drying time [min] | - | - | - | - | - | - | - | - | - | 60 | - |

The following evaluations were carried out for Examples 10 to 33, Test Examples 8 to 26, and Comparative Examples 2 to 4.

### (Test Methods)

An evaluation of whether or not each drug composition imparts stability (especially thermal stability) to the Cryj1 protein was performed by measuring the allergen activity of Cryj1, which is the allergen protein of Cedar pollen. The evaluation method will be explained below.

### (Allergen activity evaluation method)

The allergen activity of Cryj1, which is one of the primary allergens of Cedar pollen, was measured using the Cedar pollen antigen ELISA kit "Cryj1" (product of Seikagaku Biobusiness Corp.) This measurement kit is based on the sandwich ELISA procedure utilizing monoclonal antibodies (013 and 053) that are specific to Cryj1, which is one of the pollen allergens of the Japanese Cedar (*Cryptomeria japonica*), and it enables the specific measurement of Cryj1. First 20 µL of reference solution or sample was added to 100 µL of the reaction buffer of the kit, and after the first reaction was carried out at normal temperatures for 60 min, 100 µL of horseradish peroxidase (HRP)-recognition antibody solution was added so that a second reaction was carried out for 60 min. Then 100 µL of an enzyme substrate solution was added, a reaction was carried out in the dark at normal temperatures for 30 min, and finally 100µL of a reaction halting solution was added. After the reaction, the UV absorption intensity was measured at 450 nm. A calibration curve was prepared based on the absorption values of reference solutions at each Cryj1 concentration, and then the Cryj1 allergen activity (ng/mL) of each sample was measured using that curve.
In the stability and heating test the initial value of the amount of Cryj1 added to each sample was assigned a value of 100%, and the residual Cryj1 allergen activity after sampling or after heating and drying was evaluated as a percentage thereof.
The results are shown in Tables 12 to 17.

**Table 10**

| Samples | Residual allergen activity [%] | |
|---|---|---|
| | 1 week later | 2 weeks later |
| Example 1 | 92 | 69 |
| Example 2 | 81 | 59 |
| Example 3 | 80 | 25 |
| Example 4 | 67 | 47 |
| Test Example 1 | 54 | 43 |
| Test Example 2 | 16 | 2 |
| Test Example 3 | 35 | 12 |
| Test Example 4 | 29 | 5 |
| Test Example 5 | 49 | 22 |

**Table 11**

| Samples | Residual allergen activity [%] | |
|---|---|---|
| | 1 week later | 2 weeks later |
| Example 5 | 77 | 78 |
| Example 6 | 68 | 21 |
| Example 7 | 66 | 10 |
| Example 8 | 84 | 79 |
| Example 9 | 73 | 50 |
| Test Example 6 | 47 | 34 |
| Test Example 7 | 30 | 6 |
| Comparative Example 1 | 33 | 11 |

**Table 12**

| Samples | Residual allergen activity [%] |
|---|---|
| Example 10 | 63 |
| Test Example 8 | 22 |
| Test Example 9 | 46 |
| Test Example 10 | 0 |
| Comparative Example 2 | 53 |

**Table 13**

| Samples | Residual allergen activity [%] |
|---|---|
| Example 11 | 57 |
| Test Example 11 | 25 |
| Test Example 12 | 41 |
| Test Example 13 | 0 |
| Comparative Example 3 | 43 |

**Table 14**

| Samples | Residual allergen activity [%] |
|---|---|
| Example 12 | 76 |
| Test Example 14 | 28 |
| Test Example 15 | 57 |
| Test Example 16 | 63 |
| Comparative Example 4 | 43 |

**Table 15**

| Samples | Residual allergen activity [%] |
|---|---|
| Test Example 17 | 36 |
| Test Example 18 | 27 |
| Test Example 19 | 22 |
| Test Example 20 | 0 |
| Test Example 21 | 2 |
| Test Example 22 | 0 |
| Test Example 23 | 0 |
| Test Example 24 | 0 |
| Test Example 25 | 0 |
| Test Example 26 | 62 |

**Table 16**

| Samples | Residual allergen activity [%] |
|---|---|
| Example 13 | 69 |
| Example 14 | 55 |
| Example 15 | 46 |
| Example 16 | 62 |
| Example 17 | 59 |
| Example 18 | 56 |
| Example 19 | 47 |
| Example 20 | 46 |
| Example 21 | 42 |
| Example 22 | 63 |

**Table 17**

| Samples | Residual allergen activity [%] |
|---|---|
| Example 23 | 98 |
| Example 24 | 97 |
| Example 25 | 93 |
| Example 26 | 72 |
| Example 27 | 57 |
| Example 28 | 49 |
| Example 29 | 35 |
| Example 30 | 20 |
| Example 31 | 12 |
| Example 32 | 105 |
| Example 33 | 115 |

As shown in Tables 10 and 11, a high level of residual allergen activity was retained after one week in all the stabilized pharmaceutical composition of the examples that contained water and used at least one type of stabilizer selected from the group consisting of D-sorbitol, isomalt, sucrose, raffinose, sorbitan fatty acid esters, and sucrose fatty acid esters as a stabilizer.
In contrast, the residual allergen activity after one week was lower than that of the film-form preparations of the examples in all the drug compositions of the comparative examples that did not contain a stabilizer and in all the drug compositions of the test examples that contained water, but did not use at least one type of stabilizer selected from the group consisting of D-sorbitol, isomalt, sucrose, raffinose, sorbitan fatty acid esters, and sucrose fatty acid esters as a stabilizer.

Furthermore, as shown in Tables 12 to 17, a high level of residual allergen activity was retained in all the film-form preparations of the examples that contained essentially no water and had D-mannitol as the stabilizer.
In contrast, the residual allergen activity was lower than that of the film-form preparations of the examples in all the film-form preparations of the comparative examples that did not contain a stabilizer and in all the film-form preparations of the test examples that contained essentially no water and did not use D-mannitol as a stabilizer.

### INDUSTRIAL APPLICABILITY

The stabilized pharmaceutical composition of the present invention enables the production of a preparation for desensitization therapy (a liquid preparation of a stabilized pharmaceutical composition or film-form preparation) that is useful for the prevention or treatment of an allergic disease such as pollen hypersensitivity, has excellent stability of the allergen protein, and is useful for storage and transfer.

### EXPLANATION OF SYMBOLS

- 1a: D-mannitol particles
- 1b: Base material

## Claims

1. A stabilized pharmaceutical composition, comprising:
an allergen protein; and
at least one type of stabilizer selected from the group consisting of sugars, sugar alcohols, and sugar fatty acids.

2. The stabilized pharmaceutical composition according to claim 1, wherein,
water is further contained, and
the stabilizer includes at least one type selected from the group consisting of D-sorbitol, isomalt, sucrose, raffinose, sorbitan fatty acid esters, and sucrose fatty acid esters.

3. The stabilized pharmaceutical composition according to claim 1, wherein,
essentially no water is contained, and
the stabilizer includes D-mannitol.

4. A liquid preparation of a satabilized pharmaceutical composition, obtained using the stabilized pharmaceutical composition according to claim 2.

5. A film-form preparation, obtained using the stabilized pharmaceutical composition according to claim 3 which further contains a polysaccharide.

6. The film-form preparation according to claim 5, wherein
the polysaccharide includes hydroxypropyl cellulose.

7. A method for producing a film-form preparation, comprising:
preparing a liquid dispersion containing an allergen protein, hydroxypropyl cellulose, D-mannitol, and a polar organic solvent;
forming a thin film of the liquid dispersion; and
drying the thin film.
